# EUROPÄISCHE PATENTSCHRIFT

(11) **EP 2 243 437 B1**
(45) Veröffentlichungstag und Bekanntmachung des Hinweises auf die Patenterteilung: **02.03.2016**
(21) Anmeldenummer: 10004163.1
(22) Anmeldetag: 20.04.2010
(51) Int. Cl.: A61B 17/42, A61B 18/12

(54) **Hysterektomlegerät mit Uterusmanipulator**
Hysterectomy device with uterus manipulator
Appareil d'hystérectomie doté d'un manipulateur d'utérus

(30) Priorität: 24.04.2009 DE 102009018521; 24.04.2009 US 172270 P
(43) Veröffentlichungstag der Anmeldung: 27.10.2010
(73) Patentinhaber: OLYMPUS WINTER & IBE GMBH, 22045 Hamburg (DE)
(72) Erfinder: Bolz, Matthias, Dr., 22149 Hamburg (DE); Waldmann, Verena, 21423 Winsen (DE); Klöckner, Stephan, 22143 Hamburg (DE)
(74) Vertreter: Hausfeld, Norbert

(56) Entgegenhaltungen:
- US-A- 4 085 756
- US-A1- 2001 025 155

## Beschreibung

Die Erfindung betrifft ein Hysterektomiegerät, das im Wesentlichen aus einem Uterusmanipulator, einem HF-Generator und einer Schneidelektrode besteht.

Bei einer Hysterektomie wird der Uterus entfernt, wozu eine Anzahl von Verbindungen zu diesem und insbesondere das innere Ende der Vagina getrennt werden müssen. Das erfordert gesteuertes Halten und Bewegen des Uterus während der umfangreichen chirurgischen Arbeiten. Dazu dient der Uterusmanipulator, der mit einem zentralen Dorn, der üblicherweise zu besserem Halt mit einem Außengewinde ausgebildet ist, in den Uterus eingesteckt, bzw. eingeschraubt wird, um ihn zu halten.

Der Uterusmanipulator weist eine Portiokappe auf, die, die Portio überfassend, zusätzlichen Halt gibt. Der distale Rand der Portiokappe drückt zudem von innen gegen die Wand der Vagina im Bereich von deren innerem Ende. Vom Bauchraum her, also von außerhalb der Wand der Vagina, kann der Rand der Portiokappe ertastet werden und kann als Führung zum Ausführen eines sauberen Kreisschnittes verwendet werden.

Geschnitten wird dabei entweder mit einem kalten Messer oder mit einer HF-Schneidelektrode, die üblicherweise monopolar ausgebildet ist. Das hat jedoch die bekannten Nachteile der unbestimmten Stromführung von der Schneidelektrode durch den Körper bis zu einer außen am Körper angeordneten Neutralelektrode.

Hochfrequenzschnittverfahren mit mehreren monopolaren und bipolaren Elektroden zeigt die EP 1 527 743 A2.

Die DE 201 10 921 U1 zeigt einen Uterusmanipulator, der den erwähnten Schnitt mit einer über einen Umfangsteil erstreckten Portiokappe ausführt, aus der eine Elektrode vorschiebbar ist.

Die WO 03/015643 A2 erwähnt auf Seite 4 im ersten Absatz eine Portiokappe mit einer elektrischen Ringelektrode zum Schneiden durch die Wand des Uterus.

Die US 4,085,756 A zeigt ein Gerät, bestehend aus einem Uterusmanipulator und einer elektrisch schneidenden Zange, wobei beide Geräteteile an die unterschiedlichen Pole eines HF-Generators angeschlossen sind. Bei diesem Gerät können mit der elektrisch schneidenden Zange Tubenligaturen durchgeführt werden.

Die Aufgabe der vorliegenden Erfindung besteht darin, ein Hysterektomiegerät zu schaffen, mit dem der erwähnte Kreisschnitt zum Trennen des Uterus von der Vagina einfach und sicherer ausführbar ist.

Diese Aufgabe wird mit den Merkmalen des Anspruches 1 gelöst.

Erfindungsgemäß ist am Uterusmanipulator eine Elektrode ausgebildet, die zusammen mit der Schneidelektrode eine bipolare Anordnung bildet. Der Strom fließt also von der Schneidelektrode zur Elektrode am Uterusmanipulator auf kürzestem Wege. Risiken durch im Körper vagabundierende Ströme werden ausgeschlossen. Bei dieser bipolaren Elektrodenanordnung bleibt der Vorteil erhalten, dass sich der Operateur beim Schneidvorgang, der vom Bauchraum her, also von außerhalb der Vagina erfolgt, an der in der Vagina liegenden Portiokappe, bzw. an deren distalem Rand orientieren kann, um einen sauberen Kreisschnitt an der gewünschten Stelle zu erzielen. Dabei ist die Elektrode an der Portiokappe ausgebildet, also sehr nahe zu der Schneidelektrode, dennoch kann ein Kurzschluss zwischen den Elektroden sehr einfach vermeiden werden, wenn gemäß Anspruch 2 mit der Schneidelektrode in Anlage am Rand der Portiokappe gearbeitet wird, der isolierend ausgebildet ist.

Vorteilhaft sind die Merkmale des Anspruches 3 vorgesehen. Hierdurch wird erreicht, dass gezielt bestimmte Bereiche des Uterusmanipulators als Elektroden dienen und andere Bereiche isoliert sind. Die stromableitenden Elektrodenbereiche sind also genau definiert.

In der Zeichnung ist die Erfindung beispielsweise und schematisch dargestellt. Es zeigen:
- Fig. 1: einen Schnitt durch einen zu entfernenden Uterus mit erfindungsgemäßem Hysterektomiegerät und
- Fig. 2: einen Schnitt durch die in Fig. 1 dargestellte Portiokappe in anderer Ausführungsform.

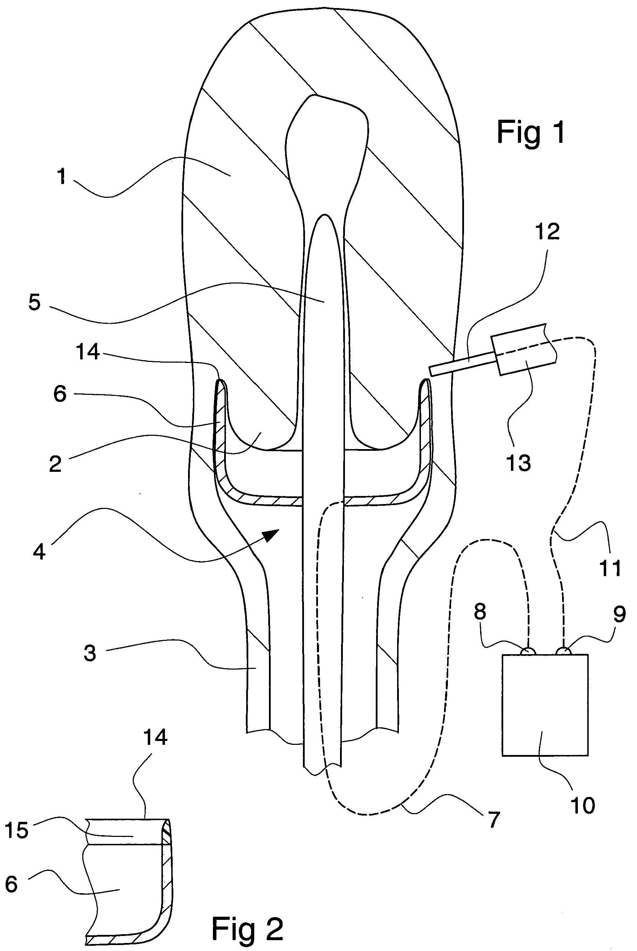

Fig. 1 zeigt einen Uterus 1 mit Portio 2, der am inneren Ende der Wand 3 der Vagina sitzt.

Im Uterus 1 ist ein Uterusmanipulator 4 platziert, der im Wesentlichen aus einem zentralen Dorn 5 und einer auf diesem befestigten Portiokappe 6 besteht.

Im dargestellten Ausführungsbeispiel ist der Dorn 5 isolierend ausgebildet und die Portiokappe 6 aus elektrisch leitfähigem Metall als Elektrode ausgebildet. Sie ist über eine Leitung 7 an einen von zwei Anschlüssen 8, 9 eines HF-Generators 10 angeschlossen, der an den Anschlüssen 8, 9 Hochfrequenzstrom zum bipolaren chirurgischen Schneiden liefert.

An den anderen Anschluss 9 des HF-Generators 10 ist mit einer Leitung 11 eine im vereinfachten Ausführungsbeispiel als Stab dargestellte Schneidelektrode 12 angeschlossen, die am distalen Ende eines Schaftes 13 sitzt, mit dem sie betätigbar ist.

Der Uterusmanipulator 4 ist so weit in die Vagina, bzw. den Uterus 1 vorgeschoben, dass der distale Rand 14 der Portiokappe 6 am äußersten inneren Ende der Wand 3 der Vagina sitzt. An dieser Stelle soll die Vagina abgeschnitten werden, um den Uterus 1 von der Vagina zu trennen. Anschließend kann der abgeschnittene Rand der Wand 3 der Vagina vernäht werden.

Der HF-Generators 10 ist mit seinen beiden Anschlüsse 8 und 9 zum bipolaren Schneiden ausgebildet. Zwischen den Elektroden 6 und 12 kann somit ein Strom fließen. Wird die Schneidelektrode 12 von dem den Uterus 1 umgebenden Bauchraum her im Endbereich der Wand 3 der Vagina eingeschnitten, so wie dies Fig. 1 zeigt, so fließt der schneidende Strom durch das Gewebe des Uterus 1 auf kürzestem Wege von der Schneidelektrode 12 zur als Elektrode dienenden Portiokappe 6.

Das Schneiden an der dargestellten Stelle, in der die Schneidelektrode unmittelbar am Rand 14 der Portiokappe schneidet, gestaltet sich für den Operateur sehr einfach, da er den Rand 14 der Portiokappe sieht, der sich nach außen abdrückt. Er kann den Rand 14 auch von außen durch die Wand 3 der Vagina hindurch ertasten. Der Operateur weiß also stets wo er ist und kann auf diese Weise gut gezielt einen Kreisschnitt rund um die Wand 3 der Vagina ausführen.

Fig. 2 zeigt in einem Ausschnitt einen Teil der Portiokappe 6 in einer Ausführungsvariante, bei soweit möglich dieselben Bezugszeichen, wie bei der Ausführungsform de Fig. 1 verwendet werden. Die Portiokappe 6 besteht wiederum aus Metall. Der distale Rand 14 der Portiokappe 6 wird jedoch von einem Isolierring 15 gebildet.

Wird die in Fig. 2 dargestellte Ausführungsform der Portiokappe 6 bei der Anordnung der Fig. 1 verwendet und steht die Schneidelektrode 12 in der in Fig. 1 dargestellten Position, so kann die Schneidelektrode 12 an den Rand 14 der Portiokappe 6 angelegt werden. Sie liegt dann an dem Isolierring 15. Es gibt keinen Kurzschluss. Dennoch fließt beim Schneiden der Strom auf kürzestem Wege um den Isolierring 15 herum zwischen der Schneidelektrode 12 und der die Gegenelektrode ausbildenden Portiokappe 6. Mit dieser Ausführungsform kann sehr einfach der Kreisschnitt mit an dem Isolierring 15 geführter Schneidelektrode 12 ausgeführt werden.

In einer weiteren Ausführungsform kann die Elektrode an dem Dorn 5 des Uterusmanipulators 4 ausgebildet sein, der z.B. im Ganzen als elektrisch leitfähige Elektrode ausgebildet sein kann, wobei die Portiokappe 6 aus Isoliermaterial besteht. Beim Schneiden mit der Schneidelektrode 12 in Position gemäß Fig. 1 fließt dann der Strom zwischen der Schneidelektrode 12 und dem Dorn 5 auf kurzem Wege. Die Schneidelektrode 12 kann dabei an der isolierenden Portiokappe geführt sein.

Das erfindungsgemäße Hysterektomiegerät wird so verwendet, dass zunächst der Uterusmanipulator 4 durch die Vagina 3 bis zum Uterus 1 vorgeschoben wird. Dabei wird der Dorn 5, wie dargestellt, durch den Cervikalkanal bis in den Uterus 1 eingeschoben, um diesen zu Manipulationszwecken sicher zu halten. Wenn zur Gewährleistung eines noch besseren Haltes auf der Außenseite des Domes 5 in seinem distalen Endbereich ein Gewinde angeordnet ist, so ist der Dorn 5 beim Einführen zu drehen.

Die Portiokappe 6 wird in der dargestellten Position, die Portio 2 umgreifend, angesetzt, um mit ihrem distalen Rand 14 so weit wie möglich am inneren Ende der Wand 3 des Uterus 1 anzuliegen. Das Einführen der Portiokappe 6 kann zugleich mit dem Einführen des Domes 5 erfolgen, wenn diese fest miteinander verbunden sind. Sind die Portiokappe 6 und der Dorn 5 lösbar miteinander verbunden, so können die beiden Teile auch getrennt eingeführt werden.

Je nach Ausführungsform des Uterusmanipulators 4 ist er in bestimmten Bereichen als Elektrode ausgebildet, beispielsweise die gesamte Portiokappe 6, wie in Fig. 16 dargestellt, oder nur der proximale Bereich der Portiokappe 6, wie in Fig. 2 dargestellt, oder z. B. nur der Dorn 5, beispielsweise nur in seinem distalen Endbereich. Eine Leitung 7 ist mit dem Elektrodenbereich des Uterusmanipulators 4 zu verbinden und mit ihrem anderen Ende an den einen Ausgang 8 des HF-Generators 10 anzuschließen.

Es ist ferner eine Schneidelektrode 12 bereitzustellen, die vorzugsweise am Ende eines Schaftes 13 sitzt. Die Schneidelektrode 12 ist mit einer Leitung 11 mit dem anderen Anschluss 9 des HF-Generators 10 zu verbinden.

Sodann ist die Schneidelektrode 12 durch einen laparoskopischen Port oder durch einen geöffneten Bauchschnitt in den Bauchraum einzubringen und außen an der Wand 3 des Uterus im Bereich der Portiokappe 6 zu platzieren, wobei die Portiokappe 6 vom Operateur von außen durch Ertasten oder durch optische Erkennung zu ermitteln ist. Unter HF-Beaufschlagung der an die beiden Leitungen 7 und 11 angeschlossenen Elektroden wird nun mit der Schneidelektrode 12 ein Kreisschnitt um die Wand 3 der Vagina herum ausgeführt. Dabei wird vorzugsweise die Schneidelektrode am Rand 14 der Portiokappe 6 angelegt gehalten, um ihr eine sichere Führung zu geben.

## Patentansprüche

1. Hysterektomiegerät mit einem Uterusmanipulator (4) und einer Schneidelektrode (12), die an einen Anschluss eines HF-Generators (10) angeschlossen ist, wobei der Uterusmanipulator (4) wenigstens bereichsweise als Elektrode (6) ausgebildet ist, welche an einen anderen Anschluss (8) des bipolar ausgebildeten HF-Generators (10) angeschlossen ist, und wobei die Schneidelektrode (12) getrennt vom Uterusmanipulator (4) bewegbar ausgebildet ist, **dadurch gekennzeichnet, dass** der Uterusmanipulator (4) eine Portiokappe (6) aufweist, an der die Elektrode ausgebildet ist.

2. Hysterektomiegerät nach Anspruch 1, **dadurch gekennzeichnet, dass** die Elektrode einen den Rand der Portiokappe (6) bildenden Isolierring (15) trägt.

3. Hysterektomiegerät nach einem der Ansprüche 1 oder 2, **dadurch gekennzeichnet, dass** die nicht als Elektrode ausgebildeten Bereiche des Uterusmanipulators (4) isolierend ausgebildet sind.

## Claims

1. A hysterectomy device having a uterus manipulator (4) and a cutting electrode (12) which is connected to a connector of an HF generator (10), wherein the uterus manipulator (4) is formed as an electrode (6) at least in sections, the electrode (6) being connected to a different connector (8) of the HF generator (10) that is formed in a bipolar manner, and wherein the cutting electrode (12) is formed such that it can be moved separately from the uterus manipulator (4),
**characterised in that**
- the uterus manipulator (4) comprises a cup pessary (6) with the electrode being formed thereon.

2. The hysterectomy device according to Claim 1, **characterised in that** the electrode carries an insulating ring (15) forming the edge of the cup pessary (6).

3. The hysterectomy device according to any one of Claims 1 or 2, **characterised in that** the areas of the uterus manipulator (4) that are not formed as an electrode are formed such that they are insulating.

## Revendications

1. Appareil d'hystérectomie doté d'un manipulateur utérin (4) et d'une électrode de résection (12) branchée sur un générateur HF (10), une partie au moins du manipulateur utérin (4) étant réalisée sous forme d'électrode (6), laquelle est branchée sur un autre branchement (8) du générateur HF (10) réalisé sous forme de générateur bipolaire, l'électrode de résection (12) étant réalisée de façon à être mobile indépendamment du manipulateur utérin (4), **caractérisé en ce que** le manipulateur utérin (4) présente un capuchon cervical (6) sur lequel est façonnée l'électrode.

2. Appareil d'hystérectomie selon la revendication 1, **caractérisé en ce que** l'électrode porte une bague isolante (15) formant le bord du capuchon cervical (6).

3. Appareil d'hystérectomie selon l'une des revendications 1 ou 2, **caractérisé en ce que** les sections du manipulateur utérin (4) ne formant pas l'électrode sont isolantes.
